# EUROPEAN PATENT SPECIFICATION

(11) **EP 3 413 867 B1**
(45) Date of publication and mention of the grant of the patent: **05.05.2021**
(21) Application number: 16804881.7
(22) Date of filing: 25.11.2016
(51) Int. Cl.: A61K 9/00, A61K 9/70

(54) **EXOTHERMIC TOPICAL DEVICES FOR DELIVERING PHARMACEUTICALS AND COSMETICS TO THE SKIN**
EXOTHERME TOPISCHE VORRICHTUNGEN ZUR ABGABE VON ARZNEIMITTELN UND KOSMETIKA AN DIE HAUT
DISPOSITIFS TOPIQUES EXOTHERMIQUES POUR ADMINISTRER DES PRODUITS PHARMACEUTIQUES ET COSMÉTIQUES À LA PEAU

(30) Priority: 30.11.2015 GB 201521111
(43) Date of publication of application: 19.12.2018
(73) Proprietor: Curapel (Scotland) Limited, Glasgow G2 4JR (GB)
(72) Inventor: LUEBCKE, Peter, Royston Hertfordshire SG8 6ND (GB); GOTHARD, Michelle, Royston Hertfordshire SG8 7XL (GB)
(74) Representative: Boult Wade Tennant LLP
(86) International application number: PCT/GB2016/053714
(87) International publication number: WO 2017/093714

(56) References cited:
- WO-A1-01/03619

## Description

### TECHNICAL FIELD

The present invention relates to the delivery of pharmaceutical agents and cosmetic agents to the skin. Provided are devices and systems for delivery of such agents. There are also provided methods of using the devices and systems, and methods for their manufacture.

### BACKGROUND

Transdermal drug delivery is increasing in prevalence and provides a practical alternative to oral and needle delivery as well as other drug delivery techniques. Transdermal delivery benefits from being non-invasive and can be self-administered often improving patient compliance. Transdermal delivery is also preferable when a patient suffers from tablet dysphagia or when needle pain and phobia needs to be avoided also preventing the generation of dangerous medical waste typical of hypodermic injections. Also, transdermally delivered drugs avoid the risk and inconvenience of intravenous therapy, it bypasses the liver in terms of first pass metabolism and elimination unlike oral delivery and avoids irritation of the GI tract, it usually provides less chance of an overdose or underdose, allows easy and rapid termination of delivery, and permits both local and systemic treatment effects and in some cases has been shown to be safer than injections¹. Since the first transdermal product in 1979 (scopolamine), the FDA has approved 35 patch products across 13 different molecules: Scopolamine (Transderm Scop - Novartis - travel sickness), fentanyl, nitroglycerin (Transderm Nitro - Novartis - angina), estradiol (Estraderm (RTM) and CombiPatch - Novartis - menopausal symptoms) norethindrone acetate, ethinylestradiol, norelgestromin, clonidine, testosterone, nicotine, lidocaine (Lidoderm (RTM) - Endo Pharmaceuticals - pain relief), prilocaine and oxybutynin.²

One well-known group of medicines that currently suffer from unwanted side-effects when taken orally is non-steroidal anti-inflammatory drugs (NSAIDs). Aspirin and Ibuprofen are examples of NSAIDs and aspirin especially is one of the most-prescribed medications worldwide, and low-dose aspirin (LDA), usually defined as 75-325 mg daily, commonly used for primary and secondary prevention of cardiovascular events and stroke³. However, the use of NSAIDs, including aspirin, is associated with risk of upper gastrointestinal mucosal damage, manifesting as peptic ulcer and/or bleeding⁴. NSAIDs, including aspirin, can also induce small-bowel injury and, now that small-bowel endoscopy is available for detection of small-bowel lesions, have become a matter of interest to gastroenterologists.⁵

Dermatological procedures such as venepuncture, heel prick, skin biopsies, shave excisions, injections, and electrosurgical, cryosurgical, and laser procedures often requiring local anaesthesia are performed frequently. Local anaesthetic preparations using patch or cream delivery systems for topical percutaneous local anaesthesia have been developed as painless alternatives to injection although usually suffer from delayed onset of anaesthesia. The potential benefits for topical delivery systems over injection include avoidance of pain increased safety, convenience of use, ease of disposal, reduced drug loading, and no tissue distention.⁶

Current nicotine patches are used to prevent cravings for cigarettes and deliver a steady dose of nicotine over the long duration wear they are recommended for. Current e-cigarettes provide a recreational delivery of nicotine more aligned with immediate nicotine satiation. Patches do not currently provide that short-term more immediate delivery.

In addition, a new trend more commonly linked to the pharmaceutical industry is emerging within the cosmetic industry with many consumers now using transdermal drug delivery patches to target cosmetic issues such as cellulite and eye contour wrinkles. The patches are increasingly desired due to their convenience with one application known to have an equivalent effect to many doses of anti-wrinkle and cellulite formulas. Additionally, one of the major benefits for cosmetics consumers is the action of targeting a specific area, such as the eye contour where sun damage leads to fine lines and wrinkles.⁷

The skin is known to be the largest organ of the human body covering an area of approximately 2m². The skin is composed of three layers: the epidermis, the dermis, and the hypodermis. The stratum corneum forms the outermost layer of the dermis and consists of 10 to 20 layers of flattened, closely packed cells without nuclei. While the stratum corneum only constitutes 10% of the entire skin, it contributes to 80% of the cutaneous barrier function.⁸ The epidermis has rapidly dividing basal cells that flatten as they move into the stratum corneum to replace cells lost from the skin's outer surface. The innermost layer is the dermis, which is a matrix of various cells (e.g. fibroblasts) including those that produce collagen and other fibre proteins. Hair follicles, sebaceous glands, and sweat glands are also part of the dermis.

By its very nature its externality makes it easily accessible. The dermis also has an excellent blood supply, and is therefore an ideal route to administer therapeutic agents. However, the main purpose of the skin is to act as a barrier, consequently rendering drug delivery into and through the skin very difficult. The passive permeation of compounds into the skin usually occurs in one of three ways. Firstly, the transappendageal pathway, e.g. through hair follicles and sweat glands, enables penetration through hair follicles but only accounts for 1-2% of the total skin surface. The remaining two routes comprise transcellular, i.e. through the cells, and paracellular, i.e. between the cells.

There has been a proliferation of techniques to enhance drug penetration through the skin from sonophoresis (ultrasound) to iontophoresis (applied electric field) often in combination with more traditional techniques such as pre-abrasion and/or chemical permeation enhancement. However, such techniques need to meet scientific, regulatory and consumer requirements to be approved and adopted within the market. Especially in the case of electromechanical systems, devices in development are more costly and complicated compared to conventional transdermal patch therapies. Typically they may rely upon electrical and mechanical components which increase the possibility for discomfort and increase potential safety risks to patients due to poor operator technique or device malfunction. In addition, effects of the device on the skin must be reversible, as any permanent damage to the stratum corneum will cause the loss of its barrier properties and hence its function as a protective organ.

More recently there has been a proliferation of research and development of what are termed 'microneedle' patches. These rely on the penetration of the stratum corneum which an array of needles typically 200-750µm in length and with a needle density of 150-650/cm². Physical penetration and creation of channels large enough to deliver large molecules such as insulin and vaccines have prompted considerable R&D expenditure and commercial activity. However microneedles, at the time of writing, have still not resulted in a market-ready product despite 10-15 years of development. This is largely due to the difficult and complex manufacturing techniques required to produce precisely controlled needle length and drug dosing per needle. In addition, Needles are prone to breakage, irritation of the skin, low dose delivery, blockage of hollow needle delivery due to choked flow from debris from the skin, bleeding and pain if needle length is too long. Microneedle patches are also limited in area which limits their suitability for large area cosmetic treatments or ones where musculoskeletal pain needs to be reduced over a large area.

Abrasive techniques, such as microdermabrasion, have been employed by dermatologists in the treatment of acne and skin blemishes and involve the removal or disruption of the upper layers of the skin to enhance the diffusion of topically applied substances. The delivery potential of skin abrasion techniques is not governed by the physicochemical properties of the drug and studies have shown that the delivery of a small molecule hydrophilic substances such as vitamin C have been enhanced⁹ as well as vaccines¹⁰ via the immunization targeting of Langerhans cells in the skin. The stratum corneum's barrier function has been reduced via several different techniques previously such as a projected stream of abrasive mineral particles, motorised devices and the motion of arrays of microabraders against the skin to enhance delivery or extraction. Such techniques rely on protrusions of known length that are shaped to avoid penetration beyond the stratum corneum.

A wide range of patches have also been produced for drug delivery. These include patches have been developed which include an anhydrous polymer layer containing or surrounding a water-soluble compound which is activated by contact with water (e.g. US6280765B1, US5817332A and US6063398A and WO0007531 A1).

Self-heating patches have also been developed. These include those based on an oxidation reaction usually that of the oxidation of iron powder (e.g. US2013345649A1, US6546281B1, WO 01/03619A1 and WO2005120472A1) and those that use an exothermic hydration reaction based on an anhydrous salt (e.g. WO2005023194A2). For example, WO 01/03619A1 describes an exothermic device for topically delivering an active agent comprising a liquid reservoir comprising water, a heating element comprising an oxidizable material, an oxygen-permeable outer-layer, an active agent, and a water-impermeable layer. Patches based on exothermic iron oxidation patches tend to be bulky, heavy and suffer from poor adhesion properties. The compositions are also prone to the generation of methane and hydrogen during long storage periods and need additional compounds, often sulphur containing, to be included that can reduce this problem. Other known problems with the development of oxidative exothermic products include the need to create an oxygen free environment during manufacture and assembly which can be expensive to create and maintain.

### SUMMARY OF THE INVENTION

The invention provides a device for delivering an agent to the skin of a subject comprising:
a. an anhydrous agent, wherein the agent is a pharmaceutical agent or a cosmetic agent,
b. an anhydrous heating component, and
c. a reservoir containing a first solvent and a second solvent, wherein the first solvent and the second solvent are different and wherein the second solvent is water;
wherein in use the first and second solvents are released from the reservoir and the second solvent hydrates the anhydrous agent to produce a solution comprising the first solvent, the second solvent and the agent, and hydrates the anhydrous heating component which reacts exothermically thereby heating the solution,
and wherein the device further comprises: (i) a backing layer, wherein the backing layer is permeable to vapour of the first solvent, (ii) a solid substrate, wherein in use a portion of the first solvent is removed from the solution by adsorption onto the solid substrate, or (iii) a copolymer, wherein in use a portion of the first solvent is separated from the solution by selective absorption by the copolymer.

The invention further provides a system comprising:
a. a device for delivering an agent to the skin of a subject comprising
   i. an anhydrous agent, wherein the agent is a pharmaceutical agent or a cosmetic agent, and
   ii. an anhydrous heating component; and
b. a reservoir containing a first solvent and a second solvent, wherein the first solvent and the second solvent are different and wherein the second solvent is water;
wherein in use the first and second solvents are released from the reservoir and the second solvent hydrates the anhydrous agent to produce a solution comprising the first solvent, the second solvent and the agent, and hydrates the anhydrous heating component which reacts exothermically thereby heating the solution,
and wherein the device further comprises: (i) a backing layer, wherein the backing layer is permeable to vapour of the first solvent, (ii) a solid substrate, wherein in use a portion of the first solvent is removed from the solution by adsorption onto the solid substrate, or (iii) a copolymer, wherein in use a portion of the first solvent is separated from the solution by selective absorption by the copolymer.

The invention provides devices and systems that enable delivery of an agent (e.g. a pharmaceutical agent or a cosmetic agent) to the skin of a subject and the transdermal delivery of an agent. The devices and systems enable localised delivery or systemic delivery of an agent. The devices and systems comprise anhydrous agents making them suitable for long-term storage (including the long-term storage of water-sensitive agents). The devices and systems, in use, produce a heated concentrated solution of the agent to be delivered promoting rapid delivery of the agent to its site of action.

Described herein is a device for delivering an agent to the skin of a subject comprising: (a) an anhydrous agent, wherein the agent is a pharmaceutical agent or a cosmetic agent; (b) an anhydrous heating component; and (c) a reservoir containing a solvent. In use solvent released from the reservoir hydrates the anhydrous agent to produce a solution and hydrates the anhydrous heating component which reacts exothermically thereby heating the solution.

Described herein is a system comprising: (a) a device for delivering an agent to the skin of a subject comprising an anhydrous agent, wherein the agent is a pharmaceutical agent or a cosmetic agent, and an anhydrous heating component; and (b) a reservoir containing a solvent. In use solvent released from the reservoir hydrates the anhydrous agent to produce a solution and hydrates the anhydrous heating component which reacts exothermically thereby heating the solution.

Preferably, in the device or system described herein the solvent is water.

Described herein is a device for delivering an agent to the skin of a subject comprising: (a) an anhydrous agent, wherein the agent is a pharmaceutical agent or a cosmetic agent; (b) an anhydrous heating component; and (c) a reservoir containing a first solvent and a second solvent (wherein the first solvent and the second solvent are different solvents). In use solvent released from the reservoir hydrates the anhydrous agent to produce a solution (comprising the first solvent, the second solvent and the agent) and hydrates the anhydrous heating component which reacts exothermically thereby heating the solution.

Described herein is a system comprising: (a) a device for delivering an agent to the skin of a subject comprising an anhydrous agent, wherein the agent is a pharmaceutical agent or a cosmetic agent, and an anhydrous heating component; and (b) a reservoir containing a first solvent and a second solvent (wherein the first solvent and the second solvent are different solvents). In use solvent released from the reservoir hydrates the anhydrous agent to produce a solution (comprising the first solvent, the second solvent and the agent) and hydrates the anhydrous heating component which reacts exothermically thereby heating the solution.

### Solvents

During use of the device or system (a portion of) the first solvent (e.g. ethanol) may be removed from the solution over time thereby concentrating the agent in the solution. At least 10%, at least 20%, at least 30%, at least 40%, at least 50%, at least 60%, at least 70%, at least 80%, at least 90%, at least 95% or at least 99% of the first solvent may be removed from the solution in the 30 minute period after solvent release from the reservoir. This removal of the first solvent may be achieved when the device is used at ambient temperature (e.g. 20°C).

The first solvent may be removed from the solution by evaporation. In this respect, the device may comprise a pervaporation membrane through which first solvent is removed from the solution.

The first solvent may be separated from the solution by selective absorption by a copolymer. The copolymer may be such a polyacrylate gel e.g. a polyacrylate gel of hydrophobic butyl acrylate and/or a polyacrylate gel of hydrophilic hydroxyethyl acrylate.

The device or system may further comprise a solid substrate, and during use of the device or system, the first solvent may be removed from the solution by adsorption onto the solid substrate. Preferably the solid substrate is activated carbon.

The first solvent may be a solvent with a lower boiling point than water and the second solvent may be water. The first solvent may be a stronger solvent for the anhydrous agent than water. The first solvent may be a co-solvent. The first solvent may be a water-miscible organic solvent. The first solvent may be ethanol, acetal (e.g. methylal and/or butylal), benzyl alcohol, dimethyl sulfoxide, ether, glycerol, glycol ether, methanol, isopropyl alcohol, 1,3-propanediol, 1-propanol, and/or propylene glycol. Preferably the first solvent is ethanol. More preferably, the first solvent is ethanol and the second solvent is water.

The second solvent is water.

The anhydrous agent may have a higher solubility in the first solvent than in the second solvent.

The reservoir may contain the first solvent and the second solvent as a mixed solvent. Alternatively, the first solvent and second solvent may be provided in separate reservoirs and then mixed together when released in use. The reservoir may further contain at least one, at least two, or at least three other solvents.

Examples of solvents suitable for use with particular pharmaceutical/cosmetic agents are provided in the table below.

| **Pharmaceutical/cosmetic agent** | **Stronger solvent(s)** | **Weaker solvent(s)** |
|---|---|---|
| Lidocaine | Ethanol or methanol | Water |
| Retinol | Ethanol, methanol or lipids | Water |
| Ibuprofen | Ethanol | Water |
| Tetracaine | Ethanol | Water |
| Nicotine | Ethanol or ether | water |
| Fentanyl | Ethanol or methanol | water |
| Testosterone | Ethanol | water |
| Oestradiol | Ethanol | Water or lipids |
| Cyclosporine | Ethanol or propylene glycol | Water |
| Aspirin | Ethanol | water |

The reservoir may further contains a skin penetration enhancer. For example, the penetration enhancer may be propylene glycol, Zemea (RTM) Propanediol, transcutol(RTM)-P, menthol, glycerol (propane-1,2,3-triol), diethylene glycol monoethyl ether, 3,7,11-Trimethyl-1,6,10-dodecatrien-3-ol, D-α-Tocopheryl polyethylene glycol 1000 succinate (vitamin E TPGS), a non-ionic surfactant, a cationic surfactant, an anionic surfactant, a terpene, a pyrrolidone, dimethyl sulfoxide, urea, a cyclic urea analogue, a phospholipid, lecithin, 1-dodecylazacycloheptan-2-one, menthol, limonene, sesquiterpene, lauric acid, myristic acid, capric acid, 4-decyloxazolidin-2-one, 1-butanol, 1-octanol, dimethyl formamide and/or MSM (methylsulfonylmethane). Preferably, the penetration enhancer is propylene glycol.

The reservoir containing the first and second solvent may be pressurised. For example, the absolute pressure may be greater than 100 kPa. A gas may be dissolved in the first and/or second solvent in the pressurised reservoir. Preferably the gas is carbon dioxide. During use of the device or system, on release of the first and second solvent from the reservoir, the gas is released from solution which facilitates the flow of the solvent from the reservoir onto the anhydrous agent and anhydrous heating component. Furthermore the release of the gas over time facilitates mixing thereby maintaining a concentration gradient of the agent across the skin.

### Anhydrous agents and anhydrous agent composites

The device or system may comprise a single anhydrous agent or may comprise at least two, at least three, at least four or at least five different anhydrous agents. Each of the anhydrous agents may be as described below.

The anhydrous agent may be a hydrophilic agent (e.g. L-ascorbic acid and/or aspirin), a lipophilic agent (e.g. a retinoid) or an amphiphilic agent. The device and system are particularly well suited to the delivery of hydrophilic agents to the skin (e.g. L-ascorbic acid and aspirin) as they can be stored under anhydrous conditions. Such agents tend to degrade when stored in the presence of water.

The anhydrous agent may be a pharmaceutical agent or a cosmetic agent. Alternatively, or additionally, the anhydrous agent may be a diagnostic agent.

The pharmaceutical agent may be an analgesic agent, an anaesthetic agent (e.g. a local anaesthetic), an anti-acne agent, an anti-allergy agent, an anti-arthritis agent, an anticancer agent, an anti-depressant, an anti-diabetic agent, an anti-infectant agent, an anti-inflammatory agent (e.g. a non-steroidal anti-inflammatory agent), an anti-hypertensive agent, anti-migraine agent, an anti-obesity agent, an antibiotic, an antiemetic, an antimicrobial, an antiviral agent (e.g. an antiretroviral agent), an appetite suppressant, a contraceptive agent, a cardiovascularly active agent, a dermatological therapeutic (e.g. an eczema therapeutic, a psoriasis therapeutic and/or an Ichthyosis therapeutic), a drug cessation agent, a fungal control agent, a hormone, an immunizing antigen, a sedative, a smoke cessation agent, a steroid (e.g. an androgen and/or an estrogen), a stimulant (e.g. caffeine and/or taurine) and/or a vaccine.

The pharmaceutical agent may be a drug that has received approval for dermal/transdermal use. For example, the pharmaceutical agent may be scopolamine, nitroglycerin, clonidine, estradiol, fentanyl, nicotine, testosterone, lidocaine with epinephrine, estradiol with norethidrone, lidocaine, ethinyl estradiol with norelgestromin, estradiol with levonorgestrel, oxybutynin, lidocaine, lidocaine with tetracaine, fentanyl HC1, methylphenidate, methyl salicylate, menthol, selegiline, rotigotine, and rivastigmine.

The pharmaceutical agent may be an agent used to target localised pain. For example, the pharmaceutical agent may be a topical non-steroidal anti-inflammatory drug (NSAID) such as naproxen, ibuprofen, indomethacin, diclofenac and ketoprofen and/or a pharmaceutically acceptable salt thereof.

The pharmaceutical agent may be used to provide local anaesthesia prior to procedures such as heel prick, cannulation, skin grafts and mole removal, local anaesthetics. Examples of such agents include amethocaine (tetracaine), lidocaine (lignocaine) and/or prilocaine.

The pharmaceutical agent may be an acne targeting agent. For example, the pharmaceutical agent may be an antibacterial, a retinoid and related preparations, such as benzoyl peroxide, isotretionoin, tretinoin and triclosan.

The pharmaceutical agent may be a dermatological disorder targeting agent such as triamcinolone acetonide (e.g. for neurodermatitis) or botulinum toxin (e.g. for axillary hyperhidrosis) or an agent that targets common skin disorders (e.g. psoriasis and/or atopic dermatitis).

The pharmaceutical agent may be an antiviral such as acyclovir, cidofovir, diphencyprone, an antiretro viral, imiquimod, interferon and/or a pharmaceutically acceptable salt thereof.

The pharmaceutical agent may be an antimitotic agent such as bleomycin, a keratolytic agent (e.g. salicylic acid) or an anti-fungal agent (e.g. terbinafine and/or itraconazole).

The cosmetic agent may be one or more of an antioxidant, an amino acid, a peptide, a polysaccharide, an oligosaccharide, a glycosaminoglycan, a proteoglycan, a non-digestible sugar, uronic acid, an anti-glycation agent, a fatty acid, a ceramide, a glycoglycerolipid, a skin lightening agent, an anti-aging agent, a film-forming agent, a retinoid, a humectant, an anti-cellulite agent, cell proliferation agent, an extracellular matrix expression stimulating agent, a vasodilatory agent, a CoQ10 quinone, a retro-photo damage compound, a fat lipolysis agent, a prebiotic, a probiotic, a stimulant (e.g. caffeine and/or taurine), an organic UV protection compound, a firming and lifting agent (e.g. Botox (RTM) or botulinum toxin) and/or a collagen enhancer (e.g. matrixyl (RTM), a palmitoyl-peptide, a palmitoyl-oligopeptide or a palmitoyl-tripeptide). Preferably, the cosmetic agent when used in the cosmetic treatment of a subject (e.g. a human) does not have a therapeutic effect on the subject.

The device may comprise the anhydrous agent in an anhydrous agent composite. Preferably the anhydrous agent composite comprises a carrier polymer. Preferably, the carrier polymer is water soluble. The anhydrous agent composite may be anhydrous fibres, anhydrous particles (or granules) or an anhydrous film. The fibres may have a average diameter of 5-500 µm, 10 to 400 µm, 25 to 300 µm, 50 to 200 µm, 75 to 100 µm, 5-250 µm, 10 to 100 µm or 25 to 50 µm. Alternatively, the fibres may have an average diameter of less than 1 µm, less than 500 nm, less than 250 nm or less than 200 nm.

The anhydrous agent may be formulated by spinning. For example, melt spinning, wet spinning, or electrospinning. In the case of wet spinning, a solvent other than water is used in order to maintain anhydrous conditions. The anhydrous agent may be formulated by extrusion e.g. hot melt extrusion.

The carrier polymer may be polyethylene oxide, polyethylene glycol, hyaluronic acid, hydroxypropyl methylcellulose, methyl cellulose, carboxymethylcellulose, hydroxymethyl cellulose, ethyl cellulose, hydroxypropyl cellulose, hydroxyethyl cellulose, sodium carboxymethyl cellulose, polymethylacrylate, polyvinylpyrollidone, cyclodextrins, chitosan, polyvinyl alcohol, pullulan, gelatin, sodium alginate, maltodextrins, silicone gel, gellan gum, iota carrageenan, kappa carrageenan, starch, modified starch, starch derivative (e.g. amylose and/or dextrin), polyacrylic acid (PAA), xanthan gum, pectin and/or guar gum. The carrier polymer may be a blend of any of the carrier polymers described herein.

The anhydrous agent composite may further comprise a plasticiser. The plasticiser may be polyethylene oxide, mannitol, glycerol, polyoxime, polyoxyethylated oil (caster oil), polyethylene glycol and/or vitamin E.

In devices that comprise more than one anhydrous agent, each agent may be stored in a spatially separate location of the device. For example, each agent may be provided in a separate anhydrous agent composite.

### Anhydrous heating components

The anhydrous heating component may comprise an anhydrous salt capable of reacting exothermically with the solvent (i.e. a solvent comprising water). The anhydrous salt may be a metal salt e.g. a metal chloride, a metal oxide and/or a metal sulphate. The anhydrous salt may be calcium chloride, magnesium sulphate, magnesium chloride, calcium oxide, barium oxide, magnesium sulphate, ferric chloride, ferrous chloride, aluminium sulphate hexahydrate and/or aluminium chloride. Preferably the anhydrous salt is calcium chloride and/or magnesium sulphate.

The anhydrous heating component may comprise an anhydrous salt capable of reacting exothermically with the solvent and a carrier polymer. Preferably the carrier polymer is water absorbing. The carrier polymer may be insoluble in water. The anhydrous heating component may be anhydrous fibres, anhydrous particles (or granules) or an anhydrous film. The fibres may have a average diameter of 5-500 µm, 10 to 400 µm, 25 to 300 µm, 50 to 200 µm, 75 to 100 µm, 5-250 µm, 10 to 100 µm or 25 to 50 µm. Alternatively, the fibres may have an average diameter of less than 1 µm, less than 500 nm, less than 250 nm or less than 200 nm.

The anhydrous heating component may be formulated by spinning. For example, melt spinning, wet spinning, or electrospinning. In the case of wet spinning, a solvent other than water is used in order to maintain anhydrous conditions. The anhydrous heating component may be formulated by extrusion e.g. hot melt extrusion.

The carrier polymer may be polyethylene oxide, polyethylene glycol, hyaluronic acid, hydroxypropyl methylcellulose, methyl cellulose, carboxymethylcellulose, hydroxymethyl cellulose, ethyl cellulose, hydroxypropyl cellulose, hydroxyethyl cellulose, sodium carboxymethyl cellulose, polymethylacrylate, polyvinylpyrollidone, cyclodextrins, chitosan, polyvinyl alcohol, pullulan, gelatin, sodium alginate, maltodextrins, silicone gel, gellan gum, iota carrageenan, kappa carrageenan, starch, modified starch, starch derivative (e.g. amylose and/or dextrin), polyacrylic acid (PAA), xanthan gum, pectin and/or guar gum. The carrier polymer may be a blend of any of the carrier polymers described herein. Preferably, the carrier polymer is a polyacrylate (e.g. sodium polyacrylate) and/or an alginate (e.g. sodium alginate).

The anhydrous heating component may further comprise a plasticiser. The plasticiser may be polyethylene oxide, mannitol, glycerol, polyoxime, polyoxyethylated oil (caster oil), polyethylene glycol and/or vitamin E.

During use of the device or system, the anhydrous heating component may form a gel on hydration. Preferably the gel is a hydrogel. The gel may comprise a polyethylene oxide, polyethylene glycol, hyaluronic acid, hydroxypropyl methylcellulose, methyl cellulose, carboxymethylcellulose, hydroxymethyl cellulose, ethyl cellulose, hydroxypropyl cellulose, hydroxyethyl cellulose, sodium carboxymethyl cellulose, polymethylacrylate, polyvinylpyrollidone, cyclodextrins, chitosan, polyvinyl alcohol, pullulan, gelatin, sodium alginate, maltodextrins, silicone gel, gellan gum, iota carrageenan, kappa carrageenan, starch, modified starch, starch derivative (e.g. amylose and/or dextrin), polyacrylic acid (PAA), xanthan gum, pectin and/or guar gum. The gel may comprise a blend of any of the carrier polymers described herein. Preferably, the gel comprises a polyacrylate (e.g. sodium polyacrylate) and/or an alginate (e.g. sodium alginate).

Preferably, the anhydrous heating component remains distinct from the anhydrous agent composite on hydration. The anhydrous heating component may be a cross-linked system e.g. a gel (as described herein). During use of the device or system, on hydration the anhydrous salt may be retained within the heating component. Preferably, substantially all of the salt is retained within the heating component. The anhydrous heating component may comprise calcium chloride and an alginate and/or pectin. Calcium chloride forms a hydrogel with alginates and pectin due, at least in part, to calcium cross linking thus retaining the calcium chloride within the heating component when the device is used. Leaching of ions (e.g. calcium ions) from the heating component may also be reduced by the incorporation of EDTA in the anhydrous heating component and/or in the anhydrous agent composite.

In addition to the heating contribution of the anhydrous salt, in use, the hydration of other components of the heating component (e.g. polyethylene glycol) and/or components of the anhydrous agent composite (e.g. polyethylene glycol) may generate heat.

### Phase Change Materials (PCMs)

The device may further comprise a phase change material (PCM). Preferably, the PCM is a blend of two or more (different) PCMs. The blend may be a eutectic a eutectic mixture. The PCM may have a melting point of 30-60°C, 35-55°C, 40-50°C or 40-45°C. The PCM may have a melting point of less than 50°C. Preferably, the PCM has a melting point of 40-45°C. The PCM has a melting point at the chosen temperature to limit the temperature rise of the solution produced by the device in use and to prolong the heating effect for as long as possible via the solid-to-liquid latent heat-absorbing phase change and then releasing the stored heat upon resolidification.

The PCM may be a fatty acid, paraffin wax and/or a salt hydrate. The PCM may be lauric acid, stearic acid, capric acid, palmitic acid and/or myristic acid. Preferably, the PCM is lauric acid and/or myristic acid. The PCM may be a eutectic mixture of lauric acid with another fatty acid e.g. stearic acid, capric acid and/or palmitic acid. The PCM may be a paraffin wax comprising hydrocarbon molecules containing 21-27 carbon atoms.

A PCM may be provided in the anhydrous agent composite and/or the anhydrous heating component.

### Other agents that may be included in the device

The device may further comprise a crystalline material capable of releasing gas on contact with the solvent. Preferably, the crystalline material is a gasified crystalline material (e.g. "popping candy"). During use of the device or system, on release of the first and second solvent from the reservoir, the crystalline material may physically rupture as it dissolves causing physical agitation of the solution in its immediate vicinity thus maintaining the concentration gradient of the agent across the skin. Additionally or alternatively gas released from solution facilitates the flow of the solvent from the reservoir onto the anhydrous agent and anhydrous heating component. Furthermore the release of the gas over time facilitates mixing thereby maintaining a concentration gradient of the agent across the skin.

Additionally or alternatively, the anhydrous agent (e.g. D-glucuronic acid, L-ascorbic acid or a suitable pharmaceutical agent) may be gasified under pressure. An anhydrous agent capable of releasing gas on contact with the solvent provides the advantages described above for the crystalline material.

The device may further comprise an anti-nucleation agent e.g. hydroxypropyl methylcellulose (HPMC), hydroxypropylcelluose (HPC), polyvinyl alcohol (PVA), polyvinylpyrrolidone (PVP), and polyethylene glycol (e.g. PEG 4000 and/or PEG 8000). The anti-nucleation agent may be provided in the anhydrous agent composite.

### Device structure

The device may be a patch (e.g. an anhydrous patch). Preferably, the device is a transdermal patch (e.g. an anhydrous transdermal patch).

The device may comprise a backing layer (or outer layer). Preferably, the backing layer is permeable to the solvent vapour (e.g. the vapour of the first solvent). The backing layer may be thermally insulating to reduce heat loss.

The device may comprise: (a) an anhydrous agent composite comprising the anhydrous agent and a carrier polymer; (b) an anhydrous heating component as described herein; and (c) a backing layer, optionally wherein the backing layer is permeable to the solvent vapour. The anhydrous heating component may form a gel on hydration.

The device may further comprise a spacer layer between the backing layer and the other components of the device (i.e. the anhydrous agent composite and anhydrous heating component). The spacer layer may comprise a porous material e.g. a mesh.

The anhydrous agent composite may be water soluble. The anhydrous agent composite may be anhydrous fibres, anhydrous particles (or granules) or an anhydrous film. The anhydrous heating component may be anhydrous fibres, anhydrous particles (or granules) or an anhydrous film.

The anhydrous agent composite and the anhydrous heating component may be provided in the device in the same layer e.g. interwoven. The anhydrous agent composite and the anhydrous heating component may form an anhydrous matrix. The anhydrous agent composite and the anhydrous heating component may be provided as a mesh. In such a mesh a proportion of the fibres comprise an anhydrous salt capable of reacting exothermically with the solvent (and, optionally a PCM) and the remainder comprise the anhydrous agent (e.g. pharmaceutical agent or cosmetic agent). The orientation of fibres in the mesh may be randomly aligned (i.e. non-woven) or may be ordered. For example, the different types of fibres may be perpendicularly aligned relative to each other. The fibres may also be arranged in sequential layers such that each layer is entirely composed of one type of fibre.

The anhydrous agent composite and the anhydrous heating component may be present in the device as granules (or particles). Preferably the granules have sufficient porosity to promote water flow through capillary action causing wicking up into and throughout the granular particle bed. The anhydrous agent composite and the anhydrous heating component may be separate and discrete from each other and are mixed in the required ratio and evenly distributed. This way the heating will be uniform throughout the active layer avoiding the formation of hot and cold regions.

The device may comprise in sequence: (a) a first layer comprising the anhydrous agent (or anhydrous agent composite); (b) a second layer comprising the anhydrous heating component; and (c) a third layer, wherein the third layer is a backing layer. Preferably, the second and third layers are permeable to vapour of the first solvent. During use of the device or system, the first layer is positioned skin side.

The anhydrous agent composite and the anhydrous heating component may be present as separate layers of film. Preferably, the anhydrous agent composite is provided as layers of dissolvable film. The layers may have a plurality of holes or channels across and through the entire thickness of these layers. To maintain strength and patch shape, layers of water-insoluble textile/fabric (e.g. having an open-weave structure) may be positioned between layers of anhydrous agent composite and layers of the anhydrous heating component. This may also help wick aqueous fluid through the multiple active-loaded and heating layers of the device.

The device may comprise a first solvent pervaporation membrane e.g. an ethanol pervaporation membrane. The backing layer may comprise the first solvent pervaporation membrane. The first solvent pervaporation membrane may comprise the backing layer.

The first solvent pervaporation membrane may be the backing layer. Preferably, the pervaporation membrane is provided between the backing layer and the layer or layers that provide the anhydrous agent composite and the anhydrous heating component.

The device may comprise an adhesive perimeter, by means of which the device is, in use, secured to the skin. Preferably the adhesive is hypoallergenic. The adhesive may provide sufficient tack to prevent loss of solution from the edges of patch but not too strong to compromise the integrity of the skin to which it is adhered. An example of a suitable adhesive is DURO-TAK(RTM). The adhesive may minimise maceration of the underlying skin, i.e. allow water loss from natural trans-epidermal water loss (TEWL).

Alternatively the device may not comprise an adhesive perimeter. Such a device may adhere to the skin due to the adhesive properties of the hydrated components in use.

The reservoir of the device may be connected to the anhydrous agent composite and the anhydrous heating component by a channel that, prior to use, is closed by a frangible seal. In use the frangible seal is broken releasing the solvent onto the anhydrous agent composite and the anhydrous heating component e.g. by the subject applying pressure to the reservoir.

The system may comprise the device provided in a first sealed (e.g. hermetically sealed) compartment of a packaging system (e.g. a blister pack) and the reservoir provided by a second spatially separated sealed (e.g. hermetically sealed) compartment of the packaging system. The first and second compartments of the packaging system are connected by a frangible seal. Preferably, the first and second compartments are connected by a channel that, prior to use, is closed by the frangible seal. In use the frangible seal is broken releasing the solvent onto the device e.g. by the subject applying pressure to the second compartment.

Alternatively, the system may comprise the device provided in a first sealed (e.g. hermetically sealed) compartment of a packaging system (e.g. a blister pack) and a physically separate reservoir (e.g. a bottle, ampoule or sachet) for use with the device.

Preferably the device or system does not comprise a preservative. The anhydrous agent and the solvent provided in the reservoir do not require a preservative; the former due to the absence of water and the latter due to the high level of a strong solvent (e.g. ethanol). In both cases the growth of bacteria, moulds and fungi during storage are inhibited by the conditions, enabling long-term storage of the device or system.

During use of the device or system, after the initial heating period, the agent may become increasingly saturated in the solution due to subsequent cooling.

The device or system may, in use, result in supersaturation of the agent in the solution on the skin of the subject. The main driving force for diffusion through the skin is the thermodynamic activity of the drug or permeant in the donor solution. This 'activity' is directly related to the concentration of this permeant as related to its saturation solubility within that solution. The closer to saturation the permeant becomes the higher its thermodynamic activity and consequently the higher the escaping tendency from the solution. Creation of supersaturated solutions can be achieved by the addition of weak or 'non' solvent to a strong (or good) solvent both of which are saturated with respect to the solute/permeant. This scenario can also be created by the loss of a stronger solvent from a mixed solvent system leaving the solute dissolved in an increasingly higher percentage of a weak solvent.

During use of the device or system, a supersaturated solution may be formed partly from the loss of a solvent, preferably a stronger solvent (e.g. ethanol), from solution and partly from the subsequent cooling of the solution following the initial heating period caused by the exothermic hydration reaction.

The device or system, in use, produces a heated solution of the agent (e.g. pharmaceutical agent or cosmetic agent). Heat assists delivery into and across the skin through a process termed 'thermophoresis' (also known as thermal poration) which is due to an applied temperature gradient, the greater mobility of actives in the aqueous hydrogel, lowering of viscosity and disruption of stratum corneum lipids. Thermophoresis results in the formation of aqueous pathways across the stratum corneum and has been used to deliver conventional drugs¹¹.

Heating stimulates thermophoresis which, on its own, promotes transdermal delivery. The device or system, in use, using this this heating effect to create a supersaturated solution by: volatilising the stronger solvent (e.g. ethanol) forcing the agent to be dissolved in a solution which contains more of the weaker solvent (e.g. water); and as the more volatile solvent (e.g. ethanol) is driven off the solution will also start to cool after the initial heating period also reducing the solubility of the agent in the now cooler and weaker solvent solution.

### Kits

Described herein is a kit comprising: (a) an abrasive pad and (b) a device or system as described herein.

The abrasive pad may comprise a penetration enhancer (or lipid barrier reducing solvent). The penetration enhancer may be menthol, linalool, ethanol, propylene glycol, deionised water and/or MSM (methylsulfonylmethane). This pad may be used to reduce the barrier function of the stratum corneum and increases the hydrophilic nature of the outer epidermis.

The kit further may further comprise a skin cream. Preferably, the cream is a barrier restoring cream e.g. a lipid emulsion. Preferably, the cream is applied by the user after the device or system has been used to deliver the agent to the subject's skin. Preferably, the cream enhances retention in the skin of the agent delivered by the device or system.

The kit may be provided in one or more sealed (e.g. hermetically sealed) compartments of a packaging system (e.g. a blister pack). The kit may comprise the following: the device comprising a reservoir (e.g. a patch) in a first sealed compartment; an abrasive pad in a second sealed compartment; and, optionally, a skin cream in a in a third sealed compartment.

Alternatively, the kit may comprise the following: the device (e.g. a patch) in a first sealed compartment; the reservoir in a second spatially separated sealed compartment; an abrasive pad in a third sealed compartment; and, optionally, a skin cream in a in a fourth sealed compartment. The first and second compartments of the packaging system may be connected by a frangible seal. Preferably, the first and second compartments are connected by a channel that, prior to use, is closed by the frangible seal. In use the frangible seal is broken releasing the solvent onto the device e.g. by the subject applying pressure to the second compartment.

### Methods of treatment and cosmetic methods

Described herein is a method of treating a subject comprising: (a) contacting a device with a solvent comprising a first solvent and a second solvent, wherein the device comprises an anhydrous pharmaceutical agent, and an anhydrous heating component, wherein the solvent hydrates the anhydrous pharmaceutical agent to produce a solution (comprising the first solvent, the second solvent and the agent) and hydrates the anhydrous heating component which reacts exothermically thereby heating the solution; and (b) contacting the skin of the subject with the device thereby delivering the agent to the skin of the subject.

Described herein is a method of treating a subject using a device or system as defined herein comprising: (a) releasing the solvent from the reservoir, wherein solvent released from the reservoir hydrates the anhydrous agent to produce a solution and hydrates the anhydrous heating component which reacts exothermically thereby heating the solution, and (b) contacting the skin of the subject with the device thereby delivering the agent to the skin of the subject.

Described herein is a method of delivering a cosmetic agent to the skin of a subject comprising: (a) contacting a device with a solvent comprising a first solvent and a second solvent, wherein the device comprises an anhydrous cosmetic agent, and an anhydrous heating component, wherein the solvent hydrates the anhydrous agent to produce a solution (comprising the first solvent, the second solvent and the agent) and hydrates the anhydrous heating component which reacts exothermically thereby heating the solution; and (b) contacting the skin of the subject with the device thereby delivering the agent to the skin of the subject.

Described herein is a method of delivering a cosmetic agent to the skin of a subject using a device or system as described herein comprising: (a) releasing the solvent from the reservoir, wherein solvent released from the reservoir hydrates the anhydrous agent to produce a solution and hydrates the anhydrous heating component which reacts exothermically thereby heating the solution; and (b) contacting the skin of the subject with the device thereby delivering the agent to the skin of the subject.

In the methods, after step (a), (a portion of) the first solvent is removed from the solution over time thereby concentrating the pharmaceutical agent or cosmetic agent in the solution.

At least 10%, at least 20%, at least 30%, at least 40%, at least 50%, at least 60%, at least 70%, at least 80%, at least 90%, at least 95% or at least 99% of the first solvent may be removed from the solution in the 30 minute period after solvent release from the reservoir (i.e. after step (a)). This removal of the first solvent may be achieved when the device is used at ambient temperature (e.g. 20°C).

The first solvent may be removed from the solution by evaporation. The evaporation of the first solvent is promoted by the exothermic reaction of the anhydrous heating component which heats the solution.

The device may comprise a backing layer and the first solvent may be removed from the solution by evaporation through the backing layer.

Additionally or alternatively, the first solvent may be removed from the solution by adsorption onto a solid substrate. Preferably, the solid substrate is activated carbon (and the first solvent is ethanol).

In the methods, after step (b), the pharmaceutical agent or cosmetic agent may become supersaturated in the solution on the skin.

In the methods, after step (b), the solution on the skin may reach a temperature of 30-60°C, 35-55°C, 40-50°C or 40-45°C. The maximum temperature of the solution on the skin may not exceed 50°C. Preferably, the solution on the skin reaches a temperature of 40-45°C. The temperature may be reached in less than 20 minutes, less than 15 minutes, less than 10 minutes or less than 5 minutes after step (a). The temperature may be maintained for 10 to 60 minutes, 15 to 45 minutes, 20 to 40 minutes or 25 to 35 minutes. These temperatures may be reached and maintained when the device or system is used at ambient temperature e.g. 20°C.

The device (e.g. patch) may be applied to the skin of the subject and left in position for 1 minute to 12 hours, 5 minutes to 2 hours, 10 minutes to 1 hour or 15-30 minutes. Preferably, the device is left in position at least until a supersaturated solution is formed on the skin of the subject.

The pharmaceutical agent or cosmetic agent may be delivered transdermally.

In the methods, any of the devices, systems or kits described herein may be used.

In the methods the subject may be an animal e.g. a mammal. Preferably the subject is a human.

### Medical uses

Described herein is an anhydrous pharmaceutical agent for use in the treatment of a subject, wherein the agent is administered to the subject by a therapeutic method comprising: (a) contacting a device with a solvent comprising a first solvent and a second solvent, wherein the device comprises an anhydrous pharmaceutical agent, and an anhydrous heating component, wherein the solvent hydrates the anhydrous pharmaceutical agent to produce a solution (comprising the first solvent, the second solvent and the agent) and hydrates the anhydrous heating component which reacts exothermically thereby heating the solution; and (b) contacting the skin of the subject with the device thereby delivering the agent to the skin of the subject.

Described herein is an anhydrous pharmaceutical agent for use in the treatment of a subject, wherein the agent is administered to the subject using a device or system as described herein, and wherein the agent is administered to the subject by a therapeutic method comprising: (a) releasing the solvent from the reservoir, wherein solvent released from the reservoir hydrates the anhydrous agent to produce a solution and hydrates the anhydrous heating component which reacts exothermically thereby heating the solution; and (b) contacting the skin of the subject with the device thereby delivering the agent to the skin of the subject.

The anhydrous pharmaceutical agent may be for use in the treatment of pain, acne, allergy, arthritis, cancer, depression, diabetes, infection, inflammation, hypertension, migraine, bacterial infection, vomiting, virus-caused disease, cardiovascular disease, eczema, psoriasis, dermal scar tissue, ichthyosis and/or fungus-caused disease. The anhydrous pharmaceutical agent may be for use in the vaccination of the subject against a pathogen.

In use, after step (a), (a portion of) the first solvent is removed from the solution over time thereby concentrating the pharmaceutical agent or cosmetic agent in the solution. At least 10%, at least 20%, at least 30%, at least 40%, at least 50%, at least 60%, at least 70%, at least 80%, at least 90%, at least 95% or at least 99% of the first solvent may be removed from the solution in the 30 minute period after solvent release from the reservoir. This removal of the first solvent may be achieved when the device is used at ambient temperature (e.g. 20°C).

The first solvent may be removed from the solution by evaporation. The evaporation of the first solvent is promoted by the exothermic reaction of the anhydrous heating component which heats the solution.

The device may comprise a backing layer and the first solvent may be removed from the solution by evaporation through the backing layer.

Additionally or alternatively, the first solvent may be removed from the solution by adsorption onto a solid substrate. Preferably, the solid substrate is activated carbon (and the first solvent is ethanol).

In use, after step (b), the pharmaceutical agent or cosmetic agent may become supersaturated in the solution on the skin.

In use, after step (b), the solution on the skin may reach a temperature of 30-60°C, 35-55°C, 40-50°C or 40-45°C. The maximum temperature of the solution on the skin may not exceed 50°C. Preferably, the solution on the skin reaches a temperature of 40-45°C. The temperature may be reached in less than 20 minutes, less than 15 minutes, less than 10 minutes or less than 5 minutes after step (a). The temperature may be maintained for 10 to 60 minutes, 15 to 45 minutes, 20 to 40 minutes or 25 to 35 minutes. These temperatures may be reached and maintained when the device or system is used at ambient temperature e.g. 20°C.

The pharmaceutical agent or cosmetic agent may be delivered transdermally.

In the medical uses, any of the devices, systems or kits described herein may be used. The subject may be an animal e.g. a mammal. Preferably the subject is a human.

### Methods of manufacture

Described herein is a method of manufacturing the device or system as described herein comprising: (a) formulating the anhydrous agent with a carrier polymer by spinning or extrusion to produce an anhydrous agent composite; and (b) preparing the anhydrous heating component. Preferably, the anhydrous heating component is prepared by spinning or extrusion. The anhydrous agent composite and the anhydrous heating component may be prepared separately and then combined as separate layers or a single layer in the manufacture of the device. Preferably the anhydrous agent composite and the anhydrous heating component are prepared in the absence of water.

Spinning may be melt spinning, wet spinning, or electrospinning. In the case of wet spinning, a solvent other than water is used in order to maintain anhydrous conditions. In the case of electrospinning, an electrospun fibre mat may be prepared using an organic solvent. The anhydrous agent may be formulated by extrusion e.g. hot melt extrusion.

The anhydrous agent composite and the anhydrous heating component may be prepared together e.g. formed of the same material. For example, the anhydrous agent composite and the anhydrous heating component may be formulated together by extrusion e.g. hot melt extrusion.

Electrospinning presents a number of advantages, in particular, the possibility to fabricate high specific surface area structures. Fibre diameters as low as 100's of nanometres can be manufactured via the electrospinning process producing fibre 'mats' that allow rapid wicking of fluids into the mat. The fibres may have an average diameter of less than 1 µm, less than 500 nm, less than 250 nm or less than 200 nm. A range of polymers can be electrospun that form soluble hydrogels such as polyvinyl alcohol (PVOH), HPMC and/or hyaluronic acid.

More conventional and lower cost extrusion (e.g. hot-melt) and other spinning techniques may be employed to create fibres with larger diameters. For example the fibres may have an average diameter of 5-500 µm, 10 to 400 µm, 25 to 300 µm, 50 to 200 µm, 75 to 100 µm, 5-250 µm, 10 to 100 µm or 25 to 50 µm. Such fibres offer a cheaper alternative to electrospinning.

The method may further comprise the step of (c) attaching the anhydrous agent composite and the anhydrous heating component to a backing layer.

In the methods, steps (a) and (b) may be performed under anhydrous conditions. Preferably, steps (a), (b) and (c) are performed under anhydrous conditions. Most preferably, all of the steps of the manufacturing method are performed under anhydrous conditions.

The method may further comprise the step of adding a reservoir containing a solvent to the device.

The method may further comprise the step of sealing the device in a packing system under anhydrous conditions.

### BRIEF DESCRIPTION OF THE DRAWINGS

The present disclosure will now be described, by way of example only, with reference to the accompanying drawings in which:
Figure 1 illustrates a device (a patch) of the invention.
Figure 2 illustrates a system of the invention.
Figure 3 illustrates the structure of the different layers of a device of the invention in use.

### DETAILED DESCRIPTION

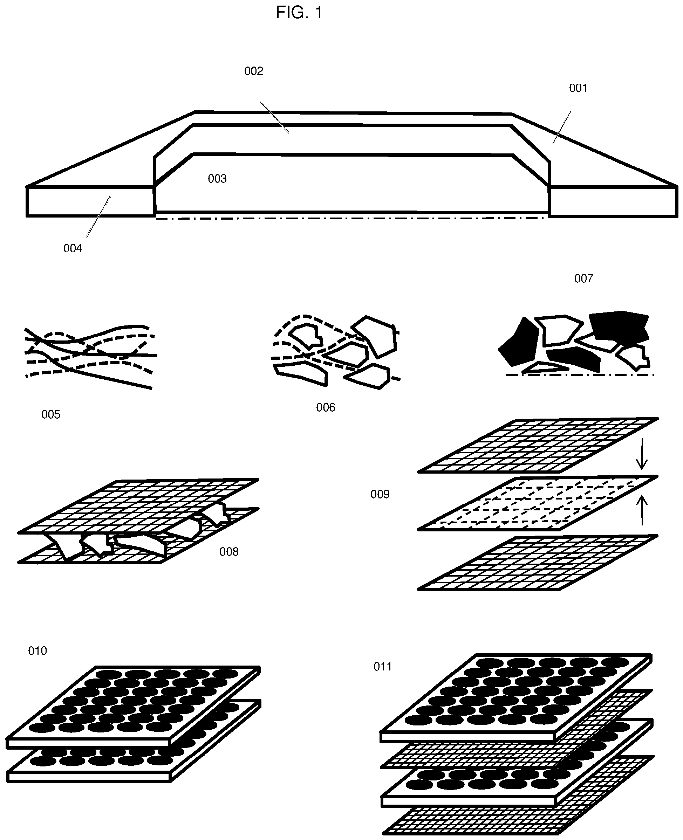

Figure 1 illustrates a device (a patch) of the invention. The patch has a textile vapour permeable backing layer (001); optionally a layer (002) that promotes loss of ethanol from the heated solution e.g. an ethanol pervaporation membrane, an activated carbon layer which may be in the form of activated carbon fibres or a gel with high ethanol affinity; an anhydrous bed of water-soluble polymer matrix carrier material (003) capable of rapidly wicking up a volume of solvent and within which a pharmaceutical agent(s) or cosmetic agent(s) is/are entrained in one component of the anhydrous bed and within another component exothermic hydration materials and phase change materials are entrained; optionally an adhesive perimeter to adhere the patch to the skin (004)

The outer visible layer (001) could be an attractive fabric that has a sensorial feel and allows escape of ethanol vapour. The loss of the latter creates the supersaturated solution of the drug/cosmetic agent pushing that substance deeper into the skin. An optional middle layer (002) could be ethanol absorbing or adsorbing such as activated carbon so preventing the need for loss of ethanol vapour from the outer surface. An adhesive perimeter (004) holds the patch in place.

Different anhydrous morphologies of the soluble polymer (003) containing the pharmaceutical/cosmetic agent and the water absorbing heating component may be manufactured (see (005) to (011)). Both components need to be able to 'wick' up the added solvent (including water). The two components may be provided interwoven as fibres, threads or strands (005). Alternatively, one component may be present as a fibre, thread or strand and the second component may be present as solid particles interspersed with amongst the fibres (006). The two components may be present as solid particles mixed together to form a granular bed (007). One component may be present as solid particles positioned between the layers of the second component present as a solid mesh (008). The two components may be present as alternating mesh layers (009). The two components may be present as alternating layers of a film or sheet similar to that manufactured as oral dissolving film whereby a degree of porosity is provided by channels through the films (010). The two components may be present, one as a permeable film/sheet and the other as a mesh (011).

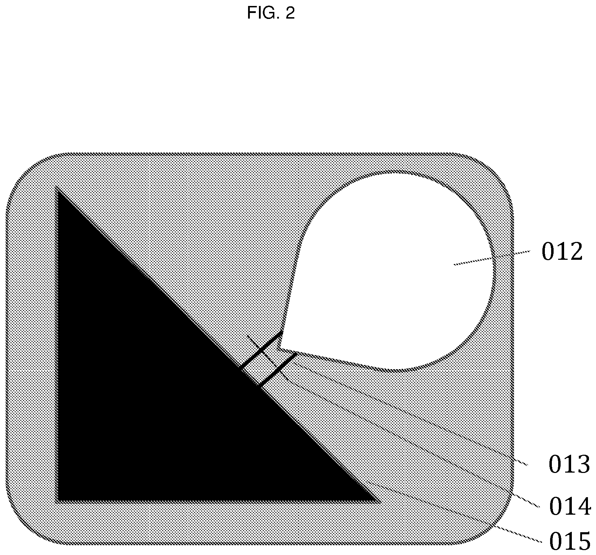

Figure 2 illustrates a system of the invention. The figure shows the spatially separated solvent reservoir (012) connected via pre-formed channel (013) which has been mechanically sealed with a frangible seal (014) that can be ruptured via firm finger pressure applied to the solvent reservoir (012). The solvent is then forced through the channel (013) into the patch compartment (015).

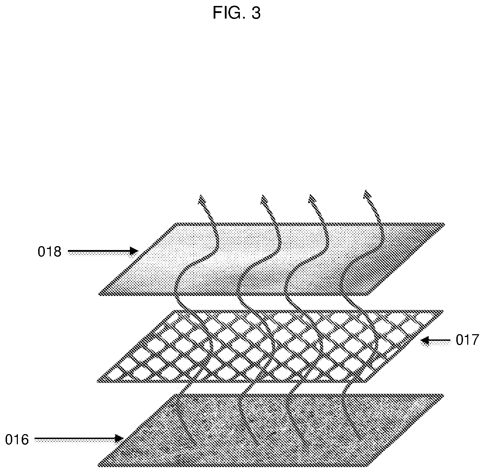

Figure 3 illustrates the structure of the different layers of a device of the invention in use. Starting with the layer proximal to the skin, there is shown a drug-loaded soluble polymer layer (i.e. produced from anhydrous agent composite on hydration) (016), an exothermic layer (produced from anhydrous heating component on hydration) (017) and an outer vapour permeable backing layer (018). Arrows show the loss of ethanol vapour when the device is in use, the ethanol vapour passing through the open weave of the exothermic layer (017) and the backing layer (018) as it is removed from the solution in the device.

### EXAMPLES

### Example 1

Tetracaine (amethocaine) is formulated with soluble polymer polyethylene glycol, vitamin E TPGS and Pluronic Lecithin organogel and hot melt extruded as fine anhydrous fibres. Separately anhydrous calcium chloride and lauric acid phase change material are blended with sodium alginate and melt extruded into fine anhydrous fibres. The fibres are combined into a non-woven mat. The mat is adhered to a tactile soft but ethanol vapour permeable fabric that forms the outer layer of the patch. An outer perimeter of skin-safe pressure sensitive adhesive such as Durotak (RTM) enables the patch to adhere to the skin. The anhydrous patch is hermetically sealed within a dedicated section of a blister pack spatially separated from a hermetically sealed measured volume of an aqueous volume of deionised water, ethanol, propylene glycol, glycerol, non-ionic surfactant Pluronic(RTM) F-127 (BASF) and penetration enhancer Transcutol(RTM) P (Gattefosse). The volume of liquid can be added to the anhydrous patch by manually squeezing the liquid compartment which forces the liquid passed a frangible barrier and through a pre-engineered channel into the patch compartment. The anhydrous fibre network wicks up the liquid. In the following seconds the active-containing fibres starts to dissolve forming a solution and the shape-retaining heating fibre network will absorb the liquid triggering an exothermic heating reaction. The patch is applied to the target area of skin promptly upon addition of the liquid.

### Example 2

A pair of patches are formed from an anhydrous composite of L-ascorbic acid co-formulated with hydroxypropyl methylcellulose and a range of molecular weights of hyaluronic acid including very low molecular weight oligomers such as tetramers as well as the monomers of hyaluronic acid, D-glucuronic acid and D-N-acetyl glucosamine. Also formulated are anti-glycation actives carnosine and arginine. The composite is melt cast into thin flexible water soluble permeable films via melt-cast manufacturing processes used in oral dissolving films with soluble polymer polyvinylpyrolidone (Tₘ=150-180°C) whose melting point is below the thermal decomposition temperature of the entrained actives such as L-ascorbic acid (200-225°C) also blended with an anti-nucleation agent such as hydroxypropyl methylcellulose (HPMC) to prevent crystallisation of the actives during formation of the supersaturated solution. The films are manufactured to have some degree of through-film porosity to allow the activating fluid to travel between layers and be wicked-up into the material. In parallel, the separate anhydrous heating element is manufactured as melt-extruded fibres formed into a porous water-wickable non-woven mat positioned as consecutive layers of which will be located between the cosmetic active containing flexible films. The fibres are formed from water-absorbing sodium polyacrylate blended with the exothermically hydrating material anhydrous magnesium sulphate and phase change eutectic mixture of lauric and capric acids. Between both the cosmetic active and heating components and the outer fabric layer is an ethanol vapour permeable layer of activated carbon that can adsorb the ethanol vapour formed due to the heating of the aqueous activating fluid. A textile outer backing material is adhered to the activated carbon layer with an attractive design of patterns and colours. The patches are housed within a hermetically sealed blister pack. A separately contained volume of an aqueous solution of deionised water, ethanol, propylene glycol and glycerol is contained within a physically separate ampoule that can be undone and added to the film/fibre composite patches. No perimeter of adhesive is used in this example; however, the patch is held in place on the skin due to the lightweight nature of the patch combined with the adhesion/tackiness created due to the formation of the gels in situ.

### Example 3

N-acetyl salicylic acid (aspirin) is co-formulated with water soluble polymer polyethylene glycol and vitamin E to lower the glass transition temperature of the PEG. The anhydrous mixture is blended and formed into a coarse grain powder of particle size in the range of 500-1000µm. Optionally, crospovidone (PVPP) may be co-formulated to cause rapid disintegration of the aspirin-containing solid particles. A separate and anhydrous heating component is similarly blended and melt-extruded to form fibres that comprise anhydrous calcium chloride, sodium alginate and lauric acid. The two anhydrous components are mixed and an aggregate of fibres and solid particles and produced which are adhered firstly to a vapour permeable activated carbon fabric layer to adsorb ethanol which in turn is attached to an outer fabric layer that can be patterned with thermochromic inks to show, by way of a colour change, an underlying temperature change as the patch heats and then cools. The fibre and granular particle material is held against the activated carbon and fabric backing by an insoluble mesh or gauze that has inter-thread gap sizes which allow liquid flow to the skin but holds in the fibres and particles in place until the time of use. An outer perimeter (∼5mm width) of dermatologically-benign adhesive holds the patch in place and prevents escape of aspirin aqueous gel formed through the hydration of each of the anhydrous components. In a separate section of the packaging a measured volume of the activating aqueous solution of deionised water, ethanol, propylene glycol, transcutol(RTM)-P is contained that can be directed to the anhydrous fabric-backed patch with a simple motion of the user such as compressing the liquid section.

### Example 4

Nicotine is co-formulated with water soluble polyvinyl alcohol polymer into a porous dissolving and liquid-wicking mesh that is positioned innermost on the skin contact side of the patch. Optionally there may be an open weave fabric layer whose thread spacing is of the order of 0.5-1.0 mm to allow maximum flow of solution and maintenance of the concentration gradient adjacent to the skin. Adjacent to the nicotine loaded mesh and on the opposite side of the nicotine mesh to the skin contact side within the patch is the exothermic heating component that is also formed into a porous liquid-wicking mesh from a blend of sodium polyacrylate, anhydrous calcium chloride and lauric acid. Still further from the skin, and forming the outermost layer, is a patterned fabric that has a soft and tactile feel that also allows loss of ethanol vapour as the patch heats and after this time over the duration of use of the patch whilst in position on the skin. The anhydrous two-part mesh system is hydrated by the addition of a precise volume of an aqueous solution of deionised water, ethanol, propylene glycol, glycerol and menthol. The aqueous solution is contained adjacent to the nicotine patch in a blister pack such that simple rupturing of a thin impermeable membrane between the patch and the solution allows flow into the patch. The liquid is then wicked up by the patch in its entirety dissolving the nicotine mesh into a solution and hydrating the heating mesh to initiate the exothermic hydration reaction. The user promptly applies the patch to the skin once wetted by the solution by simple removal from its compartment of the blister pack and applying to the skin so that the patterned fabric layer is outermost. After a period of use, the user simply removes the patch which is structurally intact due to the mechanical strength conferred by the outer fabric layer and the inner skin contacting open weave retaining layer.

### Example 5

Lidocaine is formulated with polyethylene glycol, vitamin E, vitamin E TPGS and solvent electrospun into nanofibers. Similarly, anhydrous calcium chloride and lauric acid is blended with sodium polyacrylate and solvent electrospun into nanofibers. The two fibres and interspersed to form an evenly mixed non-woven mat. The mat is backed with a layer of activated carbon fibre matting and then a patterned textile layer if adhered to the carbon fibre layer providing the outer air-facing side of the patch. A thermochromic dye is added to this outer layer to show temperature changes within the patch to the user. A 5mm width outer perimeter of hypoallergenic adhesive is located around the patch to secure it to the skin. An activating liquid comprising deionised water, ethanol, propylene glycol and transcutol(RTM)-P is hermetically sealed and adjacently located close to the lidocaine patch within the same blister packaging. Upon addition of the activating fluid to the patch the clinician removes the patch and applies it to the site where the skin procedure such as cannulation, mole removal or skin graft is about to be conducted. After approximately 15-20 minutes the patch has heated, shown by the change in colour of thermochromic ink, and then cooled shown by a reversion of colour to the original colour, the clinician can remove the patch in readiness for conducting the procedure.

### Example 6

Retinoic acid is co-formulated with polyethylene glycol, pluronic lecitihin organogel and vitamin E TPGS into melt extruded fibres of the order of 10's or 100's microns diameter. Co-located and interwoven into a non-woven mat are melt-extruded fibres of sodium alginate, anhydrous magnesium sulphate and a phase change eutectic mixture of lauric (m.pt 42.6°C) and myristic acid (m.pt. 52.2°C). The mat is backed by an ethanol vapour permeable textile layer that is patterned with thermochromic ink. Separately and adjacently located in a hermetically sealed compartment is a measured volume of the aqueous activating fluid comprising deionised water, ethanol and Zemea (RTM) propanediol and urea. The barrier between the fluid and the patches is easily rupturable by concerted applied pressure to the fluid chamber and the fluid flows into the patch-containing compartment. No perimeter of adhesive is needed as the patch is lightweight and the addition of the aqueous activating fluid creates a tacky gel that adheres to the skin. The patches may be left in position even after cooling which enhances the level of saturation and may even be left on overnight to allow time-dependent diffusion into the skin.

### REFERENCES

¹ Comparative Safety of Testosterone Dosage Forms, JAMA Intern Med. May 11, 2015.
² Gordon, R.D. & Peterson, T.A., 2003, Four myths about transdermal drug delivery, Drug Deliv. Technol., 3, 1-7.
³ Patrono C, Garcia Rodriguez LA, Landolfi R, Baigent C: Low-dose aspirin for the prevention of atherothrombosis. N Engl J Med 2005, 353:2373-2383
⁴ Sakamoto C, Sugano K, Ota S, Sakaki N, Takahashi S, Yoshida Y, Tsukui T, Osawa H, Sakurai Y, Yoshino J, Mizokami Y, Mine T, Arakawa T, Kuwayama H, Saigenji K, Yakabi K, Chiba T, Shimosegawa T, Sheehan JE, Perez-Gutthann S, Yamaguchi T, Kaufman DW, Sato T, Kubota K, Terano A: Case-control study on the association of upper gastrointestinal bleeding and nonsteroidal anti-inflammatory drugs in Japan. Eur J Clin Pharmaco/2006, 62:765-772
⁵ Shishido T, Oka S, Tanaka S, Aoyama T, Watari I, Imagawa H, Yoshida S, Chayama K: Diagnostic yield of capsule endoscopy vs. double-balloon endoscopy for patients who have undergone total enteroscopy with obscure gastrointestinal bleeding. Hepatogastroenterology 2012, 59:955-959.
⁶ McCafferty DF, Woolfson AD. New patch delivery system for percutaneous local anaesthesia. Br J Anaesth 1993;71:370-4.
⁷ CosmeticsDesign.com, Louise Prance, 03-Apr-2007 (Related topics: Skin Care, Market Trends)
⁸ Polla, Penetration of Cosmetics through the stratum corneum, Cosmetics & Toiletries Magazine, 128(2), Feb 2013.
⁹ Lee et al, 2001, Lasers and microdermabrasion enhance and control topical delivery of vitamin C, J. Invest. Dermatol., 121, 1118.
¹⁰ Mikszta, J.A., Britingham, J.M., Alarcon, J., Pettis, R.J. and Dekker, J.P. (2001) "Applicator having abraded surface coated with substance to be applied", Patent (Serial Number WO 01/89622 A1).
¹¹ Sintov et al, 2003, radiofrequency driven skin microchanneling as a new way for electrically assited transdermal delivery of hydrophilic drugs, J Cotrol Rel, 89, 311-320.

## Claims

1. A device for delivering an agent to the skin of a subject comprising:
a. an anhydrous agent, wherein the agent is a pharmaceutical agent or a cosmetic agent,
b. an anhydrous heating component, and
c. a reservoir containing a first solvent and a second solvent, wherein the first solvent and the second solvent are different and wherein the second solvent is water;
wherein in use the first and second solvents are released from the reservoir and the second solvent hydrates the anhydrous agent to produce a solution comprising the first solvent, the second solvent and the agent, and hydrates the anhydrous heating component which reacts exothermically thereby heating the solution,
and wherein the device further comprises: (i) a backing layer, wherein the backing layer is permeable to vapour of the first solvent, (ii) a solid substrate, wherein in use a portion of the first solvent is removed from the solution by adsorption onto the solid substrate, or (iii) a copolymer, wherein in use a portion of the first solvent is separated from the solution by selective absorption by the copolymer.

2. A system comprising:
a. a device for delivering an agent to the skin of a subject comprising
i. an anhydrous agent, wherein the agent is a pharmaceutical agent or a cosmetic agent, and
ii. an anhydrous heating component; and
b. a reservoir containing a first solvent and a second solvent, wherein the first solvent and the second solvent are different and wherein the second solvent is water;
wherein in use the first and second solvents are released from the reservoir and the second solvent hydrates the anhydrous agent to produce a solution comprising the first solvent, the second solvent and the agent, and hydrates the anhydrous heating component which reacts exothermically thereby heating the solution,
and wherein the device further comprises: (i) a backing layer, wherein the backing layer is permeable to vapour of the first solvent, (ii) a solid substrate, wherein in use a portion of the first solvent is removed from the solution by adsorption onto the solid substrate, or (iii) a copolymer, wherein in use a portion of the first solvent is separated from the solution by selective absorption by the copolymer.

3. The device or system of claim 1 or claim 2, wherein the device further comprises:
a. (i) a backing layer, wherein the backing layer is permeable to vapour of the first solvent, and (ii) a solid substrate, wherein in use a portion of the first solvent is removed from the solution by adsorption onto the solid substrate;
b. (i) a backing layer, wherein the backing layer is permeable to vapour of the first solvent, and (ii) a copolymer, wherein in use a portion of the first solvent is separated from the solution by selective absorption by the copolymer;
c. (i) a solid substrate, wherein in use a portion of the first solvent is removed from the solution by adsorption onto the solid substrate, and (ii) a copolymer, wherein in use a portion of the first solvent is separated from the solution by selective absorption by the copolymer; or
d. (i) a backing layer, wherein the backing layer is permeable to vapour of the first solvent, (ii) a solid substrate, wherein in use a portion of the first solvent is removed from the solution by adsorption onto the solid substrate, and (iii) a copolymer, wherein in use a portion of the first solvent is separated from the solution by selective absorption by the copolymer.

4. The device or system of any one of claims 1 to 3, wherein in use the first solvent is removed from the solution by evaporation.

5. The device or system of any one of claims 1 to 4, wherein the solid substrate is activated carbon.

6. The device or system of any one of claims 1 to 5, wherein:
a. the first solvent is a solvent with a lower boiling point than water; and/or
b. the first solvent is a stronger solvent for the anhydrous agent than water; and/or
c. (i) the first solvent is a co-solvent, (ii) the first solvent is a water-miscible organic solvent, or (iii) the first solvent is ethanol.

7. The device or system of any one of claims 1 to 6, wherein the anhydrous agent has a higher solubility in the first solvent than in the second solvent.

8. The device or system of any one of claims 1 to 7, wherein the reservoir contains the first solvent and the second solvent as a mixed solvent.

9. The device or system of any one of claims 1 to 8, wherein the reservoir containing the first and second solvent is pressurised, optionally wherein a gas is dissolved in the first and/or second solvent, optionally wherein the gas is carbon dioxide.

10. The device or system of any one of claims 1 to 9, wherein:
a. the anhydrous agent is provided in fibres and/or granules; and/or
b. the anhydrous agent is formulated with a carrier polymer and/or a plasticiser, optionally wherein the carrier polymer is a soluble polymer; and optionally wherein the device comprises the anhydrous agent in an anhydrous agent composite and wherein the anhydrous agent composite further comprises a carrier polymer; and/or
c. the anhydrous agent is a hydrophilic agent, a lipophilic agent or an amphiphilic agent; and/or
d. the anhydrous heating component comprises an anhydrous salt capable of reacting exothermically with the solvent; optionally wherein the anhydrous salt is calcium chloride, magnesium sulphate, magnesium chloride, calcium oxide, barium oxide, magnesium sulphate, ferric chloride, ferrous chloride, aluminium sulphate hexahydrate and/or aluminium chloride; and/or
e. the anhydrous heating component forms a gel on hydration, optionally wherein the gel is a hydrogel.

11. The device or system of any one of claims 1 to 10, wherein the device further comprises a phase change material (PCM); optionally wherein the PCM is a fatty acid, paraffin wax and/or a salt hydrate.

12. The device or system of any one of claims 1 to 11, wherein the device comprises:
a. an anhydrous agent composite comprising the anhydrous agent and a carrier polymer,
b. the anhydrous heating component, and
c. a backing layer, optionally wherein the backing layer is permeable to the vapour of the first solvent;
optionally wherein the anhydrous heating component forms a gel on hydration; and/or optionally wherein the anhydrous agent composite and the anhydrous heating component are interwoven.

13. The device or system of claims 1 to 12, wherein the device comprises in sequence:
a. a first layer comprising the anhydrous agent,
b. a second layer comprising the anhydrous heating component, and
c. a third layer, wherein the third layer is a backing layer,
optionally wherein the second and third layers are permeable to vapour of the first solvent.

14. The device or system of any one of claims 1 to 13, wherein the device is a patch, optionally wherein the device is a transdermal patch.

## Patentansprüche

1. Vorrichtung zum Abgeben eines Mittels an die Haut eines Individuums, umfassend:
a. ein wasserfreies Mittel, wobei das Mittel ein pharmazeutischer Wirkstoff oder ein kosmetisches Mittel ist,
b. eine wasserfreie Heizkomponente, und
c. ein Reservoir, welches ein erstes Lösungsmittel und ein zweites Lösungsmittel enthält, wobei das erste Lösungsmittel und das zweite Lösungsmittel verschieden sind und wobei das zweite Lösungsmittel Wasser ist;
wobei bei Verwendung das erste und zweite Lösungsmittel aus dem Reservoir freigesetzt werden und das zweite Lösungsmittel das wasserfreie Mittel hydratisiert, um eine Lösung umfassend das erste Lösungsmittel, das zweite Lösungsmittel und das Mittel herzustellen, und die wasserfreie Heizkomponente hydratisiert, welche exotherm reagiert und dabei die Lösung aufheizt,
und wobei die Vorrichtung ferner umfasst: (i) eine Trägerschicht, wobei die Trägerschicht für Dampf des ersten Lösungsmittels permeabel ist, (ii) ein festes Substrat, wobei bei Verwendung ein Teil des ersten Lösungsmittels aus der Lösung durch Adsorption auf das feste Substrat entfernt wird, oder (iii) ein Copolymer, wobei bei Verwendung ein Teil des ersten Lösungsmittels von der Lösung durch selektive Absorption durch das Copolymer abgetrennt wird.

2. System, umfassend:
a. eine Vorrichtung zum Abgeben eines Mittels an die Haut eines Individuums, umfassend:
i. ein wasserfreies Mittel, wobei das Mittel ein pharmazeutischer Wirkstoff oder ein kosmetisches Mittel ist, und
ii. eine wasserfreie Heizkomponente, und
b. ein Reservoir, welches ein erstes Lösungsmittel und ein zweites Lösungsmittel enthält, wobei das erste Lösungsmittel und das zweite Lösungsmittel verschieden sind und wobei das zweite Lösungsmittel Wasser ist;
wobei bei Verwendung das erste und zweite Lösungsmittel aus dem Reservoir freigesetzt werden und das zweite Lösungsmittel das wasserfreie Mittel hydratisiert, um eine Lösung umfassend das erste Lösungsmittel, das zweite Lösungsmittel und das Mittel herzustellen, und die wasserfreie Heizkomponente hydratisiert, welche exotherm reagiert und dabei die Lösung aufheizt,
und wobei die Vorrichtung ferner umfasst: (i) eine Trägerschicht, wobei die Trägerschicht für Dampf des ersten Lösungsmittels permeabel ist, (ii) ein festes Substrat, wobei bei Verwendung ein Teil des ersten Lösungsmittels aus der Lösung durch Adsorption auf das feste Substrat entfernt wird, oder (iii) ein Copolymer, wobei bei Verwendung ein Teil des ersten Lösungsmittels von der Lösung durch selektive Absorption durch das Copolymer abgetrennt wird.

3. Vorrichtung oder System nach Anspruch 1 oder Anspruch 2, wobei die Vorrichtung ferner umfasst:
a. (i) eine Trägerschicht, wobei die Trägerschicht für Dampf des ersten Lösungsmittels permeabel ist, und (ii) ein festes Substrat, wobei bei Verwendung ein Teil des ersten Lösungsmittels durch Adsorption auf das feste Substrat aus der Lösung entfernt wird;
b. (i) eine Trägerschicht, wobei die Trägerschicht für Dampf des ersten Lösungsmittels permeabel ist, und (ii) ein Copolymer, wobei bei Verwendung ein Teil des ersten Lösungsmittels durch selektive Absorption durch das Copolymer aus der Lösung entfernt wird;
c. (i) ein festes Substrat, wobei bei Verwendung ein Teil des ersten Lösungsmittels aus der Lösung durch Adsorption auf das feste Substrat entfernt wird, und (ii) ein Copolymer, wobei bei Verwendung ein Teil des ersten Lösungsmittels durch selektive Absorption durch das Copolymer aus der Lösung entfernt wird; oder
d. (i) eine Trägerschicht, wobei die Trägerschicht für Dampf des ersten Lösungsmittels permeabel ist, (ii) ein festes Substrat, wobei bei Verwendung ein Teil des ersten Lösungsmittels aus der Lösung durch Adsorption auf das feste Substrat entfernt wird, und (iii) ein Copolymer, wobei bei Verwendung ein Teil des ersten Lösungsmittels aus der Lösung durch selektive Absorption durch das Copolymer abgetrennt wird.

4. Vorrichtung oder System nach einem der Ansprüche 1 bis 3, wobei bei Verwendung das erste Lösungsmittel aus der Lösung durch Verdampfen entfernt wird.

5. Vorrichtung oder System nach einem der Ansprüche 1 bis 4, wobei das feste Substrat aktivierter Kohlenstoff ist.

6. Vorrichtung oder System nach einem der Ansprüche 1 bis 5, wobei:
a. das erste Lösungsmittel ein Lösungsmittel mit einem niedrigeren Siedepunkt als Wasser ist; und/oder
b. das erste Lösungsmittel ein stärkeres Lösungsmittel für das wasserfreie Mittel als Wasser ist; und/oder
c. (i) das erste Lösungsmittel ein Co-Lösungsmittel ist, (ii) das erste Lösungsmittel ein mit Wasser mischbares organisches Lösungsmittel ist, oder (iii) das erste Lösungsmittel Ethanol ist.

7. Vorrichtung oder System nach einem der Ansprüche 1 bis 6, wobei das wasserfreie Mittel eine höhere Löslichkeit in dem ersten Lösungsmittel als in dem zweiten Lösungsmittel aufweist.

8. Vorrichtung oder System nach einem der Ansprüche 1 bis 7, wobei das Reservoir das erste Lösungsmittel und das zweite Lösungsmittel als ein gemischtes Lösungsmittel enthält.

9. Vorrichtung oder System nach einem der Ansprüche 1 bis 8, wobei das Reservoir, welches das erste und zweite Lösungsmittel enthält, unter Druck gesetzt ist, gegebenenfalls wobei ein Gas in dem ersten und/oder zweiten Lösungsmittel gelöst ist, gegebenenfalls wobei das Gas Kohlenstoffdioxid ist.

10. Vorrichtung oder System nach einem der Ansprüche 1 bis 9, wobei:
a. das wasserfreie Mittel in Fasern und/oder Granulaten bereitgestellt ist; und/oder
b. das wasserfreue Mittel mit einem Trägerpolymer und/oder einem Weichmacher formuliert ist, gegebenenfalls wobei das Trägerpolymer ein lösliches Polymer ist; und gegebenenfalls wobei die Vorrichtung das wasserfreie Mittel in einem Komposit des wasserfreien Mittels enthält, und wobei das Komposit des wasserfreien Mittels ferner ein Trägerpolymer umfasst; und/oder
c. das wasserfreie Mittel ein hydrophiles Mittel, ein lipophiles Mittel oder ein amphiphiles Mittel ist; und/oder
d. die wasserfreie Heizkomponente ein wasserfreies Salz umfasst, welches mit dem Lösungsmittel exotherm reagieren kann; gegebenenfalls wobei das wasserfreie Salz Calciumchlorid, Magnesiumsulfat, Magnesiumchlorid, Calciumoxid, Bariumoxid, Magnesiumsulfat, Ferrichlorid, Ferrochlorid, Aluminiumsulfat-Hexahydrat und/oder Aluminiumchlorid ist; und/oder
e. die wasserfreie Heizkompenente ein Gel bei Hydratation bildet, gegebenenfalls wobei das Gel ein Hydrogel ist.

11. Vorrichtung oder System nach einem der Ansprüche 1 bis 10, wobei die Vorrichtung ferner ein Phasenwechselmaterial (PCM) umfasst, gegebenenfalls wobei das PCM eine Fettsäure, ein Paraffinwachs und/oder ein Salzhydrat ist.

12. Vorrichtung oder System nach einem der Ansprüche 1 bis 11, wobei die Vorrichtung umfasst:
a. ein Komposit des wasserfreien Mittels, umfassend das wasserfreie Mittel und ein Trägerpolymer,
b. die wasserfreie Heizkomponente, und
c. eine Trägerschicht, gegebenenfalls wobei die Trägerschicht für Dampf des ersten Lösungsmittels permeabel ist;
gegebenenfalls wobei die wasserfreie Heizkomponente ein Gel bei Hydratation bildet; und/oder gegebenenfalls wobei das Komposit des wasserfreien Mittels und die wasserfreie Heizkomponente miteinander verwoben sind.

13. Vorrichtung oder System nach einem der Ansprüche 1 bis 12, wobei die Vorrichtung in Reihe umfasst:
a. eine erste Schicht, umfassend das wasserfreie Mittel,
b. eine zweite Schicht, umfassend die wasserfreie Heizkomponente, und
c. eine dritte Schicht, wobei die dritte Schicht eine Trägerschicht ist, gegebenenfalls wobei die zweite und die dritte Schicht für Dampf des ersten Lösungsmittels permeabel sind.

14. Vorrichtung oder System nach einem der Ansprüche 1 bis 13, wobei die Vorrichtung ein Pflaster ist, gegebenenfalls wobei die Vorrichtung ein transdermales Pflaster ist.

## Revendications

1. Dispositif pour administrer un agent à la peau d'un sujet comprenant :
a. un agent anhydre, dans lequel l'agent est un agent pharmaceutique ou un agent cosmétique,
b. un composant chauffant anhydre, et
c. un réservoir contenant un premier solvant et un second solvant, dans lequel le premier solvant et le second solvant sont différents et dans lequel le second solvant est l'eau ;
dans lequel, en utilisation, les premier et second solvants sont libérés depuis le réservoir et le second solvant hydrate l'agent anhydre pour produire une solution comprenant le premier solvant, le second solvant et l'agent, et hydrate le composant chauffant anhydre qui réagit de manière exothermique, en chauffant ainsi la solution,
et dans lequel le dispositif comprend en outre : (i) une couche de support, dans lequel la couche de support est perméable à la vapeur du premier solvant, (ii) un substrat solide, dans lequel en utilisation une partie du premier solvant est éliminée de la solution par adsorption sur le substrat solide, ou (iii) un copolymère, dans lequel en utilisation une partie du premier solvant est séparée de la solution par absorption sélective par le copolymère.

2. Système comprenant :
a. un dispositif pour administrer un agent à la peau d'un sujet comprenant
i. un agent anhydre, dans lequel l'agent est un agent pharmaceutique ou un agent cosmétique, et
ii. un composant chauffant anhydre ; et
b. un réservoir contenant un premier solvant et un second solvant, dans lequel le premier solvant et le second solvant sont différents et dans lequel le second solvant est l'eau ;
dans lequel, en utilisation, les premier et second solvants sont libérés depuis le réservoir et le second solvant hydrate l'agent anhydre pour produire une solution comprenant le premier solvant, le second solvant et l'agent, et hydrate le composant chauffant anhydre qui réagit de manière exothermique, en chauffant ainsi la solution,
et dans lequel le dispositif comprend en outre : (i) une couche de support, dans lequel la couche de support est perméable à la vapeur du premier solvant, (ii) un substrat solide, dans lequel en utilisation une partie du premier solvant est éliminée de la solution par adsorption sur le substrat solide, ou (iii) un copolymère, dans lequel en utilisation une partie du premier solvant est séparée de la solution par absorption sélective par le copolymère.

3. Dispositif ou système selon la revendication 1 ou la revendication 2, dans lequel le dispositif comprend en outre :
a. (i) une couche de support, dans lequel la couche de support est perméable à la vapeur du premier solvant, et (ii) un substrat solide, dans lequel en utilisation une partie du premier solvant est éliminée de la solution par adsorption sur le substrat solide ;
b. (i) une couche de support, dans lequel la couche de support est perméable à la vapeur du premier solvant, et (ii) un copolymère, dans lequel, en utilisation, une partie du premier solvant est séparée de la solution par absorption sélective par le copolymère ;
c. (i) un substrat solide, dans lequel en utilisation une partie du premier solvant est éliminée de la solution par adsorption sur le substrat solide, et (ii) un copolymère, dans lequel en utilisation une partie du premier solvant est séparée de la solution par absorption sélective par le copolymère ; ou
d. (i) une couche de support, dans lequel la couche de support est perméable à la vapeur du premier solvant, (ii) un substrat solide, dans lequel en utilisation une partie du premier solvant est éliminée de la solution par adsorption sur le substrat solide, et (iii) un copolymère, dans lequel en utilisation une partie du premier solvant est séparée de la solution par absorption sélective par le copolymère.

4. Dispositif ou système selon l'une quelconque des revendications 1 à 3, dans lequel, en utilisation, le premier solvant est éliminé de la solution par évaporation.

5. Dispositif ou système selon l'une quelconque des revendications 1 à 4, dans lequel le substrat solide est du charbon actif.

6. Dispositif ou système selon l'une quelconque des revendications 1 à 5, dans lequel :
a. le premier solvant est un solvant avec un point d'ébullition inférieur à l'eau ; et/ou
b. le premier solvant est un solvant plus fort pour l'agent anhydre que l'eau ; et/ou
c. (i) le premier solvant est un co-solvant, (ii) le premier solvant est un solvant organique miscible à l'eau, ou (iii) le premier solvant est l'éthanol.

7. Dispositif ou système selon l'une quelconque des revendications 1 à 6, dans lequel l'agent anhydre a une solubilité plus élevée dans le premier solvant que dans le second solvant.

8. Dispositif ou système selon l'une quelconque des revendications 1 à 7, dans lequel le réservoir contient le premier solvant et le second solvant sous la forme d'un solvant mixte.

9. Dispositif ou système selon l'une quelconque des revendications 1 à 8, dans lequel le réservoir contenant le premier et le second solvant est mis sous pression, facultativement dans lequel un gaz est dissous dans le premier et/ou second solvant, facultativement dans lequel le gaz est du dioxyde de carbone.

10. Dispositif ou système selon l'une quelconque des revendications 1 à 9, dans lequel :
a. l'agent anhydre est fourni sous forme de fibres et/ou de granulés ; et/ou
b. l'agent anhydre est formulé avec un polymère porteur et/ou un plastifiant, facultativement dans lequel le polymère porteur est un polymère soluble ; et facultativement dans lequel le dispositif comprend l'agent anhydre dans un composite d'agent anhydre et dans lequel le composite d'agent anhydre comprend en outre un polymère porteur ; et/ou
c. l'agent anhydre est un agent hydrophile, un agent lipophile ou un agent amphiphile ; et/ou
d. le composant chauffant anhydre comprend un sel anhydre capable de réagir de manière exothermique avec le solvant ; facultativement dans lequel le sel anhydre est le chlorure de calcium, le sulfate de magnésium, le chlorure de magnésium, l'oxyde de calcium, l'oxyde de baryum, le sulfate de magnésium, le chlorure ferrique, le chlorure ferreux, l'hexahydrate de sulfate d'aluminium et/ou le chlorure d'aluminium ; et/ou
e. le composant chauffant anhydre forme un gel par hydratation, facultativement dans lequel le gel est un hydrogel.

11. Dispositif ou système selon l'une quelconque des revendications 1 à 10, dans lequel le dispositif comprend en outre un matériau à changement de phase (PCM) ; facultativement dans lequel le PCM est un acide gras, une cire de paraffine et/ou un hydrate de sel.

12. Dispositif ou système selon l'une quelconque des revendications 1 à 11, dans lequel le dispositif comprend :
a. un composite d'agent anhydre comprenant l'agent anhydre et un polymère porteur,
b. le composant chauffant anhydre, et
c. une couche de support, facultativement dans lequel la couche de support est perméable à la vapeur du premier solvant ;
facultativement dans lequel le composant chauffant anhydre forme un gel par hydratation ; et/ou facultativement dans lequel le composite d'agent anhydre et le composant de chauffage anhydre sont entrelacés.

13. Dispositif ou système selon les revendications 1 à 12, dans lequel le dispositif comprend en séquence :
a. une première couche comprenant l'agent anhydre,
b. une deuxième couche comprenant le composant chauffant anhydre, et
c. une troisième couche, la troisième couche étant une couche de support,
facultativement dans lequel les deuxième et troisième couches sont perméables à la vapeur du premier solvant.

14. Dispositif ou système selon l'une quelconque des revendications 1 à 13, dans lequel le dispositif est un timbre, facultativement dans lequel le dispositif est un timbre transdermique.
